## Europäisches Patentamt

## European Patent Office

## Office européen des brevets

(11) Publication number: **0 060 103**
**B1**

## EUROPEAN PATENT SPECIFICATION

(45) Date of publication of patent specification: **17.04.85**

(51) Int. Cl.⁴: **C 07 C 1/20,** C 07 C 11/02

(21) Application number: **82301129.1**

(22) Date of filing: **05.03.82**

(54) **Process for manufacturing ethylene.**

(30) Priority: **05.03.81 US 240738**

(43) Date of publication of application:
**15.09.82 Bulletin 82/37**

(45) Publication of the grant of the patent:
**17.04.85 Bulletin 85/16**

(84) Designated Contracting States:
**BE DE FR GB IT NL**

(56) References cited:
**DE-A-2 615 150**
**US-A-4 148 835**

(73) Proprietor: **MOBIL OIL CORPORATION**
**150 East 42nd Street**
**New York New York 10017 (US)**

(72) Inventor: **Lee, Wooyoung**
**4 Cohasset Lane**
**Cherry Lane New Jersey (US)**
Inventor: **Shinnar, Reuel**
**110 Ash Drive Great Neck Estates**
**Great Neck New York (US)**
Inventor: **Yurchak, Sergei**
**211 Timber Jump Lane**
**Media Pennsylvania (US)**

(74) Representative: **Cooper, John Anthony et al**
**Mobil Court 3 Clements Inn**
**London WC2A 2EB (GB)**

Courier Press, Leamington Spa, England.

## Description

This invention relates to the catalytic conversion of methanol into a hydrocarbon mixture of high ethylene content.

A remarkable growth in the production of synthetic fibers, plastics and petrochemicals has taken place in recent decades. This growth, to a very large extent, has been supported and encouraged by an expanding supply of inexpensive petrochemical raw materials such as ethylene, propylene and other olefins. The principal raw material for ethylene at the present time is petroleum naphtha, which is steam cracked to produce a mixture of products from which ethylene is recovered. A large fraction of this ethylene is used for the manufacture of polyethylene and styrene.

It has been shown in recent years that methanol and dimethyl ether, which may be derived from coal and from natural gas, are converted to a mixture of hydrocarbons, including ethylene by catalytic contact with various zeolites, exemplified by HZSM-5. The reaction is highly exothermic, and the olefins initially formed tend to undergo further reaction.

Various aspects of the conversion of methanol or dimethyl ether into light olefins, especially ethylene, are described in the patent literature. For example, the production of olefins from aliphatic ethers by catalytic conversion with, for example a HZSM-5 zeolite catalyst is described in U.S. Patent 3,894,106; a two-stage conversion of a lower alcohol into olefins or into gasoline, which employs a tubular reactor for the second stage, is described in U.S. Patent 4,058,576; the use of diluents to dissipate heat in a two-stage conversion of methanol into gasoline is described in U.S. Patent 3,931,349; a process for obtaining increased yields of ethylene by converting methanol over a ZSM-5 catalyst by operating with 2—20 mols of steam diluent per mol of methanol feed is described in U.S. Patent 4,083,889; a process in which dimethyl ether is converted over a zeolite like ZSM-5 with high selectivity into olefins at 5 to 25% conversion, and at high space velocity, is described in U.S. Patent 4,052,479; the conversion of methanol, dimethyl ether, or mixtures thereof, selectively into ethylene and propylene in a continuous heat balanced cyclic process over such zeolites as erionite, chabazite, zeolite T, and ZK-5 is described in U.S. Patent 4,229,608; and the conversion of alcohols and/or ethers having no more than 4 carbon atoms over ZSM-5 type zeolites at low severity into an olefinic product aromatisable by contact with a ZSM-5 type zeolite is described in German Offenlegungsschrift 2,615,150.

Whereas the prior art discloses, broadly, that ethylene selectivity is enhanced by partial conversion of the methanol or dimethyl ether feed, it is well recognised in the art that such an expedient entails a large economic penalty because the cost of methanol dictates that the unconverted reactant must be recovered and recycled. Furthermore, the art is also aware that operating with a diluent such as steam enhances selectivity for ethylene and assists in control of reactor temperature. However, the addition of steam to the feed of necessity further dilutes unconverted feed and increases the cost of its recovery.

The present invention seeks to provide an energy efficient process for the manufacture of ethylene with high selectivity from methanol or dimethyl ether or mixtures thereof.

According to the present invention, there is provided a process for the manufacture of ethylene which comprises the steps of

(i) catalytically converting from 35 to 85 mol% of a feed comprising methanol, dimethyl ether or a mixture thereof into a product mixture;

(ii) condensing the product mixture from step (i) into an aqueous layer, a liquid hydrocarbon layer and a gaseous layer;

(iii) recovering ethylene from the gaseous layer;

(iv) recovering methanol and dimethyl ether from the aqueous layer;

(v) recycling methanol and dimethyl ether recovered in step (iv) to catalytic conversion step (i), characterized in that methanol and dimethyl ether are recovered from the aqueous layer in step (iv) by volatilization by direct or indirect contact with steam having a pressure of from 205 to 1480 kPa.

The utilization of low pressure steam according to the invention is an effective way to economically take advantage of the improved selectivity for ethylene which results from partial conversion of the feed. Low pressure steam is particularly effective when partial conversion is practiced together with steam dilution. It is an advantage of this invention that the process integrates very well with a methanol plant in which the carbon monoxide and hydrogen raw materials are provided by a Lurgi dry ash coal gasifier, since the latter gasifier is known to produce excess low pressure steam.

The catalytic conversion of the invention may be considered as the result of completely dehydrating methanol to form methylene fragments which combine to form ethylene, propylene and higher olefins, accompanied by secondary reactions which form paraffins and aromatic hydrocarbons. Under favorable conditions, substantially no decomposition of methanol to carbon monoxide occurs, and only trace amounts of oxygenated compounds other than water are formed.

The effluent from the catalytic conversion step consists of hydrocarbons including ethylene, water, and unconverted methanol and dimethyl ether. In addition to ethylene, some light paraffins and hydrocarbons having from three to about 10 carbon atoms will also be present. In general, more severe reaction conditions result in higher conversions in the range of 35 to about 85 mol% of reactant and produce more higher molecular weight hydrocarbons and less ethylene. In any event, cooling and condensing the total effluent from the catalytic conversion step yields three separate phases, a first, aqueous phase which contains a large fraction of the unreacted methanol; a second, liquid hydrocarbon phase that contains $C_3+$ hydrocarbons; and a third, gaseous phase that contains a large fraction, for example more than 90%, of the ethylene produced together with a substantial amount of other light hydrocarbons and unconverted dimethyl ether. Trace amounts of oxygenates that may form in the reactor, such as acetone and acetic acid, are concentrated in the aqueous phase. These trace by-products are largely recovered from the aqueous phase together with the unreacted methanol and recycled to the reactor, thus reducing to a minimum any ecological problem that might be encountered in their disposal. Also, the relative volume of the first, aqueous layer is increased when the conversion feed is diluted with steam and this increased volume favors partitioning of unreacted methanol into the first, aqueous phase and of unconverted dimethyl ether into the third, gaseous phase.

Steam dilution of the feed may be achieved by combining the methanol/dimethyl ether feed with steam, preferably steam having a pressure of 205 to 1480 kPa, or at least some of the required steam diluent may already be mixed with the methanol/dimethyl ether recycled to the conversion step. In either case, the total amount of diluting steam is preferably from 0.25 to 5.0 mols, more preferably from 0.5 to 2.5 mols per mol of methanol or methanol equivalent of dimethyl ether (one methanol equivalent of dimethyl ether being considered as one half mol of dimethyl ether monohydrate i.e. $CH_3OH \rightleftharpoons 1/2CH_3OCH_3 \cdot H_2O$).

According to a preferred feature of the invention, the third, gaseous phase, after further compression, is contacted with methanol to selectively sorb unreacted dimethyl ether. The methanol sorbent, charged with dimethyl ether, may then be recycled to the catalytic conversion step together with fresh methanol, recycled methanol and dimethyl ether, and where appropriate steam diluent. Alternatively, the gaseous phase may be contacted with water in order to selectively remove dimethyl ether.

The catalyst used in the conversion step may be any heterogeneous catalyst effective for converting methanol to hydrocarbons including ethylene. Known catalysts for this purpose include crystalline zeolites that have an effective pore diameter of at least 5 Angstroms and that are reasonably stable under the reaction conditions and in the presence of steam, such as hydrogen mordenite and zeolite T.

Preferred catalysts, however, are crystalline zeolites having a silica to alumina mole ratio of at least 12 and a Constraint Index of from 1 to 12, especially zeolites ZSM-5, ZSM-11, ZSM-12, ZSM-23, ZSM-35 and ZSM-38. ZSM-5 is particularly preferred. The meaning and significance of 'Constraint Index' and the method of its determination, are fully described in, for example U.S. Patent 3,907,915.

It may be desirable to incorporate the crystalline zeolites into another material resistant to the temperature and other conditions employed in the process. Such materials include synthetic or naturally occurring substances as well as inorganic materials such as clay, silica and/or metal oxides. In addition to such materials, the zeolite may be composited with a porous matrix material, such as alumina, silica-alumina, silica-magnesia, silica-zirconia, silica-thoria, silica-berylia, silica-titania as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix on an anhydrous basis may vary widely, with the zeolite content preferably ranging from 5 to 99 percent by weight and more preferably from 10 to 80 percent by weight of the dry composite.

The catalytic conversion is suitably carried out at a temperature of 260°C to 480°C, preferably from 260°C to 370°C, at a methanol weight hourly space velocity (WHSV) of from 0.5 to 5.0, and preferably with the catalyst maintained in a tubular reactor or as a fluidized bed. The conversion pressure is preferably from 170 to 620 kPa.

The present invention will now be described in greater detail by way of example only with reference to the accompanying drawing, which is a flow diagram of the process.

Referring to the drawing, fresh methanol (which may contain a small amount, for example up to 20 weight%, of a higher alcohol such as ethanol) is passed via line 1 to line 4 where it is combined with recovered methanol and dimethyl ether and with steam via lines 2 and 3. The combined feed in line 4, which includes the required amount of steam diluent, is passed to heat exchanger 5 where it is brought to reactor inlet temperature and where the pressure is adjusted, for example, to about 375 kPa. This feed stream is then passed via line 6 to reactor 7 containing a suitable conversion catalyst, where it is partially converted into hydrocarbons. The effluent from the reactor, which comprises unreacted methanol and dimethyl ether, ethylene and other hydrocarbons, and steam is passed via line 8 to a heat exchanger 11 wherein it is cooled, for example to about 95°C. Coolant for the reactor is supplied via line 9 and is removed via line 10. The coolant may be water in which case the reactor 7 serves to generate high pressure steam, that is steam having a pressure above about 4240 kPa. Whereas heat exchangers 5 and 11 are shown as two separate units, it is possible to operate with a single heat exchanger in which case the hot effluent stream 8 is utilized to preheat the mixed feed from line 4, after which the feed may be brought to reactor, inlet temperature with a trim heater (not shown).

The total effluent from the reactor 7, which has been cooled by heat exchange, is then passed via line 12 to a condenser 13 which serves to remove further amounts of heat and liquify a substantial fraction of the total effluent stream. The condensed products and gas are passed via line 14 to a separator 15 in which the mixture is resolved into three distinct phases. The total reactor effluent passed via line 14 to the separator 15 may, for example, have the following composition in weight%;

| Methanol | 8.7 |
|---|---|
| Dimethyl Ether | 8.1 |
| Water | 70.0 |
| $CO$, $CO_2$, $H_2$ Total | <0.1 |
| Methane | 0.1 |
| Ethane | <0.1 |
| Ethylene | 3.3 |
| Propane | 0.6 |
| Propylene | 2.1 |
| Isobutane | 0.6 |
| n-butane | 0.2 |
| Butenes | 1.0 |
| $C_5+$ | 5.1 |

The lowermost layer in separator 15 is the aqueous layer made up of the water produced by the conversion of methanol to hydrocarbons, the water produced by condensation of steam diluent, and unreacted methanol, with some dimethyl ether. The second layer, which appears between the aqueous layer and the gas phase, consists of hydrocarbons comprising propylene, propane and higher molecular weight aliphatic and aromatic hydrocarbons. The uppermost, gaseous, layer consists of light hydrocarbons and most of the ethylene saturated with water vapor and a substantial fraction of unconverted dimethyl ether.

The first, aqueous layer is withdrawn via line 46 and passed via pump 27 through line 28. The flow in line 28 is partitioned into lines 29 and 35, by partitioning means not shown, such that the water required for steam dilution is largely passed via line 29 to evaporator 30. Evaporation of the unreacted methanol, dimethyl ether and water in evaporator 30 is effected with low pressure steam supplied by line 32 with condensate being withdrawn by line 31, the hot vapors passing via lines 33 and 34, and then via line 3, into common feed line 4. The water to be rejected, representing essentially the water produced by the conversion of the feed to hydrocarbons, is passed as that portion of the aqueous condensate diverted through lines 35 and 36 to stripper 37. The stripper is operated by low pressure steam injected via line 38 which effectively recovers the unreacted methanol and dimethyl ether which pass via lines 39, 40, 43 and 44 to common feed line 4. Some recycle in the stripper may be provided via line 41 to improve efficiency. Waste water, substantially free of organic matter, is discharged via line 42.

The third, gaseous layer in the separator, which contains substantially all of the ethylene produced together with other light hydrocarbons, trace amounts of $CO$, $CO_2$, $H_2$, and a relatively large amount of dimethyl ether is withdrawn via line 16, compressed to about 1480 kPa by compressor 17, and passed to an absorber section 19 where it is scrubbed with methanol introduced via line 21. Methanol serves as an unusually effective sorbent for dimethyl ether and also serves to dry the ethylene. Used sorbent is withdrawn via line 25 and passed via lines 26 and 2 to contribute to the feed mixture in line 4, all without separation of sorbed dimethyl ether and water. The partially purified ethylene stream withdrawn via line 20 from the dimethyl ether absorber 19 is passed via line 20 to a gas plant 22. Ethylene of the desired purity is recovered from the gas plant via line 23 and heavier hydrocarbons such as propylene and butenes are withdrawn from the gas plant via line 24. The gas plant also serves to remove $CO$, $CO_2$, $H_2$, $CH_4$ and $C_2H_6$. The stream from line 24 and the second, liquid hydrocarbon layer in separator 15 which is withdrawn via line 45 are passed to downstream $C_3+$ recovery units or to other units for further processing. The $C_3+$ hydrocarbons may be separated into LPG and a gasoline fraction, for example.

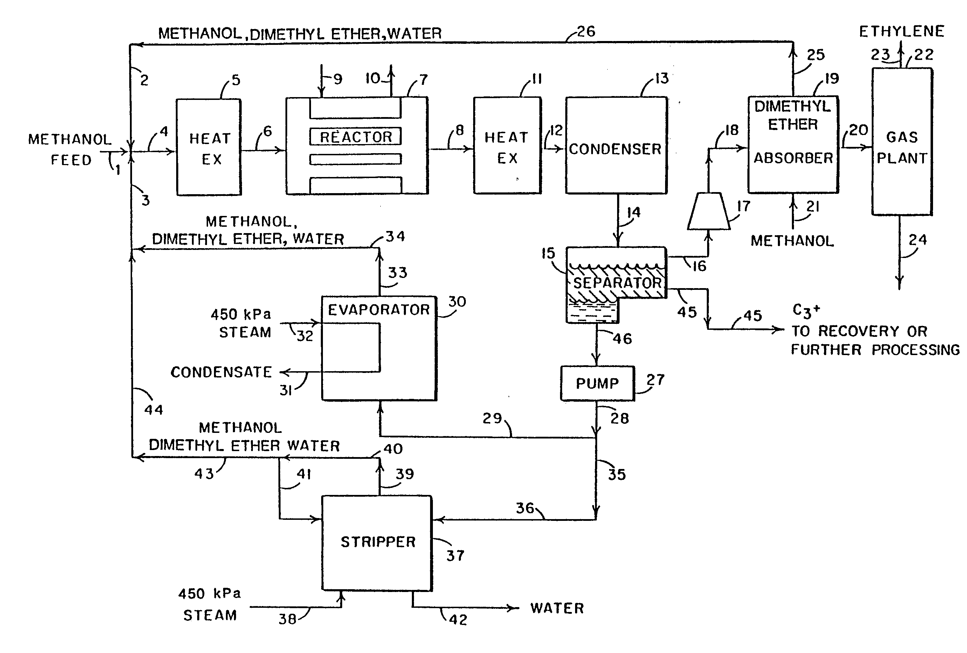

The reactor 7, shown in Figure 1, may be a multistage fixed bed reactor with interstage cooling. However, temperature control of the catalytic conversion can be more easily and more reliably achieved with either a fluidized bed or a tubular reactor, and for this reason the fluidized bed and tubular reactors are preferred. Further, the mixed feed in line 4 may be contacted with an acidic inorganic oxide, for example gamma alumina, under conditions effective to equilibrate methanol and dimethyl ether, at a temperature in the range of from 260 to 425°C and then the equilibrated mixture passed to reactor 7. This two-stage conversion permits partial removal of the heat of reaction prior to the catalytic conversion to hydrocarbons, which then becomes less exothermic. Still further, evaporator 30 may be replaced by a suitable stripper and the absorption unit 19 may be operated with captive methanol regenerated by low pressure steam.

**Claims**

1. A process for the manufacture of ethylene which comprises the steps of
(i) catalytically converting from 35 to 85 mol% of a feed comprising methanol, dimethyl ether or a mixture thereof into a product mixture;

(ii) condensing the product mixture from step (i) into an aqueous layer, a liquid hydrocarbon layer and a gaseous layer;

(iii) recovering ethylene from the gaseous layer;

(iv) recovering methanol and dimethyl ether from the aqueous layer;

(v) recycling methanol and dimethyl ether recovered in step (iv) to catalytic conversion step (i), characterised in that methanol and dimethyl ether are recovered from the aqueous layer in step (iv) by volatilization by direct or indirect contact with steam having a pressure of from 205 to 1480 kPa.

2. A process according to claim 1, wherein the feed to conversion step (i) contains from 0.25 to 5.0 mols of diluting steam per mol of methanol or methanol equivalent of dimethyl ether.

3. A process according to claim 2, wherein the amount of diluting steam is from 0.5 to 2.5 mols per mol of methanol or methanol equivalent of dimethyl ether.

4. A process according to claim 2 or claim 3, wherein at least part of the diluting steam is entrained with the methanol from step (iv).

5. A process according to any one of claims 2 to 4, wherein at least part of the diluting steam is added to the feed as steam having a pressure from 205 to 1480 kPa.

6. A process according to any one of claims 1 to 5, wherein the gaseous layer is contacted with methanol to recover dimethyl ether.

7. A process according to any one of claims 1 to 6, wherein the conversion catalyst is a crystalline zeolite having an effective pore diameter of about 5 Angstroms.

8. A process according to any one of claims 1 to 6, wherein the zeolite has a silica to alumina ratio of at least 12 and a constraint index of 1 to 12.

9. A process according to any one of claims 1 to 6, wherein the zeolite is ZSM-5, ZSM-11, ZSM-12, ZSM-23 or ZSM-38.

### Patentansprüche

1. Verfahren zur Herstellung von Ethylen, das die Stufen umfaßt des

(i) katalytischen Umwandelns von 35 bis 85 Mol% eines Einsatzproduktes, das Methanol, Dimethyläther oder eine Mischung davon umfaßt, zu einer Produktmischung;

(ii) Kondensierens der Produktmischung aus Stufe (i) zu einer wäßrigen Schicht, einer flüssigen Kohlenwasserstoffschicht und einer gasförmigen Schicht;

(iii) Gewinnens von Ethylen aus der gasförmigen Schicht;

(iv) Gewinnens von Methanol und Dimethyläther aus der wäßrigen Schicht;

(v) Zurückführens des in Stufe (iv) gewonnenen Methanols und Dimethyläthers in die Stufe (i) der katalytischen Umwandlung, dadurch gekennzeichnet, daß Methanol und Dimethyläther aus der wäßrigen Schicht in Stufe (iv) durch Vordampfen durch direkten oder indirekten Kontakt mit Dampf mit einem Druck von 205 bis 1480 kPa wiedergewonnen werden.

2. Verfahren nach Anspruch 1, bei dem das Einsatzprodukt der Umwandlungsstufe (i) von 0,25 bis 5,0 Mol Verdünnungsdampf pro Mol Methanol oder Methanoläquivalent des Dimethyläthers enthält.

3. Verfahren nach Anspruch 2, bei dem die Menge des Verdünnungsdampfes von 0,5 bis 2,5 Mol pro Mol Methanol oder Methanoläquivalent des Dimethyläthers beträgt.

4. Verfahren nach Anspruch 2 oder Anspruch 3, bei dem wenigstens ein Teil des Verdünnungsdampfes mit dem Methanol aus Stufe (iv) mitgeführt wird.

5. Verfahren nach irgendeinem der Ansprüche 2 bis 4, bei dem wenigstens ein Teil des Verdünnungsdampfes dem Einsatzprodukt als Dampf mit einem Druck von 205 bis 1480 kPa zugesetzt wird.

6. Verfahren nach irgendeinem der Ansprüche 1 bis 5, bei dem die gasförmige Schicht zur Wiedergewinnung von Dimethyläther mit Methanol kontaktiert wird.

7. Verfahren nach irgendeinem der Ansprüche 1 bis 6, bei dem der Umwandlungskatalysator ein kristalliner Zeolith mit einem effektiven Porendurchmesser von etwa 5 Angström ist.

8. Verfahren nach irgendeinem der Ansprüche 1 bis 6, bei dem der Zeolith ein Siliciumdioxid/Aluminiumoxid-Verhältnis von wenigstens 12 und einen Grenzwertindex (constraint index) von 1 bis 12 aufweist.

9. Verfahren nach irgendeinem der Ansprüche 1 bis 6, bei dem der Zeolith ZSM-5, ZSM-11, ZSM-12, ZSM-23 oder ZSM-38 ist.

### Revendications

1. Un procédé pour la fabrication d'éthylène qui comprend les étapes suivantes:

(i) conversion catalytique de 35 à 85 moles% d'une charge consistant en méthanol, éther diméthylique ou leurs mélanges en un mélange de produits;

(ii) condensation du mélange produit de l'étape (i) en une couche aqueous, une couche d'hydrocarbures liquides et une couche gazeuse;

(iii) récupération de l'éthylène de la couche gazeuse;

(iv) récupération du méthanol et de l'éther diméthylique de la couche aqueuse;

(v) recyclage du méthanol et de l'éther diméthylique récupérés dans l'étape (iv) à l'étape de conversion catalytique (i), caractérisé en ce que le méthanol de l'éther diméthylique sont récupérés de la couche aqueuse dans l'étape (iv) par volatilisation par contact direct ou indirect avec de la vapeur ayant une pression de 205 à 1 480 kPa.

2. Procédé selon la revendication 1, dans lequel l'étape de conversion (i) contient de 0,25 à 5,0 moles de vapeur de dilution par mole de méthanol ou d'équivalent méthanol d'éther diméthylique.

3. Un procédé selon la revendication 2, dans lequel la quantité de vapeur de dilution est de 0,5 à 2,5 moles par mole de méthanol ou d'équivalent méthanol d'éther diméthylique.

4. Un procédé selon la revendication 2 ou 3, dans lequel une partie au moins de la vapeur de dilution est entraînée avec le méthanol provenant de l'étape (iv).

5. Un procédé selon l'une quelconque des revendications 2 à 4, dans lequel une partie au moins de la vapeur de dilution est ajoutée à la charge sous forme de vapeur ayant une pression de 205 à 1 480 kPa.

6. Un procédé selon l'une quelconque des revendications 1 à 5, dans lequel la couche gazeuse est mise en contact avec le méthanol pour récupérer l'éther diméthylique.

7. Un procédé selon l'une quelconque des revendications 1 à 6, dans lequel le catalyseur de conversion est une zéolite cristalline ayant un diamètre efficace de pores d'environ 5 Å.

8. Un procédé selon l'une quelconque des revendications 1 à 6, dans lequel la zéolite a un rapport silice/alumine d'au moins 12 et un indice de contrainte de 1 à 12.

9. Un procédé selon l'une quelconque des revendications 1 à 6, dans lequel la zéolite est la ZSM-5, la ZSM-11, la ZSM-12, la ZSM-23 ou la ZSM-38.

METHANOL, DIMETHYL ETHER, WATER  26

ETHYLENE  23  22

METHANOL FEED

HEAT EX  5

REACTOR  7

HEAT EX  11

CONDENSER  13

DIMETHYL ETHER ABSORBER  19

GAS PLANT

METHANOL, DIMETHYL ETHER, WATER  34

METHANOL  21

EVAPORATOR  30

SEPARATOR  15

450 kPa STEAM  32

CONDENSATE  31

C₃⁺ TO RECOVERY OR FURTHER PROCESSING  45

PUMP  27

METHANOL DIMETHYL ETHER WATER

STRIPPER

450 kPa STEAM  38

WATER  42

0 060 103